(19) Europäisches Patentamt
European Patent Office
Office européen des brevets

(11) **EP 4 390 941 A1**

(12) **EUROPEAN PATENT APPLICATION**

(43) Date of publication:
**26.06.2024 Bulletin 2024/26**

(21) Application number: **22215219.1**

(22) Date of filing: **20.12.2022**

(51) International Patent Classification (IPC):
**G16C 60/00** $^{(2019.01)}$

(52) Cooperative Patent Classification (CPC):
**G16C 60/00; G06N 3/086;** G06N 3/044;
G06N 3/0464; G06N 3/048; G06N 3/088;
G06N 3/09; G06N 3/092

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Borealis AG**
**1020 Vienna (AT)**

(72) Inventors:
• **TORRES, Juan Pablo**
**4021 Linz (AT)**

• **JERABEK, Michael**
**4021 Linz (AT)**
• **RUEDA, Federico**
**CP 7600 Buenos Aires (AR)**
• **KIEHAS, Florian**
**4040 Linz (AT)**
• **REITER, Martin**
**4040 Linz (AT)**

(74) Representative: **Ter Meer Steinmeister & Partner**
**Patentanwälte mbB**
**Nymphenburger Straße 4**
**80335 München (DE)**

(54) **A COMPUTER-IMPLEMENTED METHOD FOR OUTPUTTING PARAMETER VALUES DESCRIBING AT LEAST AN ELASTOPLASTIC MECHANICAL RESPONSE OF ONE OR MORE MATERIALS**

(57)    The invention disclosed herein relates to a computer-implemented method for outputting parameter values describing at least an elastoplastic mechanical response of one or more materials, most preferably one or more polymers. The invention further relates to a non-transitory computer readable storage medium for tangibly storing computer program instructions capable of being executed by a processor, the computer program instructions defining the steps of one or more steps of the computer-implemented method for outputting parameter values as described herein.

Fig. 1

EP 4 390 941 A1

## Description

Field of the invention

The invention disclosed herein relates to a computer-implemented method for outputting parameter values describing at least an elastoplastic mechanical response of one or more materials, most preferably one or more polymers. The invention further relates to a non-transitory computer readable storage medium for tangibly storing computer program instructions capable of being executed by a processor, the computer program instructions defining the steps of one or more steps of the computer-implemented method for outputting parameter values as described herein.

More specific, it is introduced herewith a methodology to extract material properties from convoluted information sources such as one or more force-displacement signals obtained in an impact test, such as an instrumented Charpy; and/or an instrumented Izod test; and/or an instrumented puncture test; and/or an instrumented falling weight test or any other mechanical impact test involving dynamic loading of an arbitrary test specimen.

Technical Background

In modem supply chains, like the ones used in the automotive industry, original equipment manufacturers, OEMs, and their suppliers require and exploit intrinsic material property data to design and manufacture parts and components of their products. In this context, the generation of material property data that can describe the material behavior under one or more impact loading conditions is increasingly being demanded, especially for higher-end applications.

WO 2021/110 690 A1 discloses a method for determining material properties from an image of a foam sample in order to reduce testing efforts. Here, a structural feature, especially at the microscopic level, *i.e.* the microstructure, such as a pore, a wall, a strut, a node, a cell, is measured from the image and this measured structural feature is provided to a material model. The material model is a data-driven model, such as a trained machine learning model, that associates the structural features with material properties without knowledge of laws of physics. Here, structural features are directly measured from images. Such measurements require additional (expensive) equipment that increases the complexity of the method. In addition, it is necessary to transform the results of the measurement process and its errors to be suitable as input parameters. Such a transformation reduces accuracy and further increases the complexity of the method. No intrinsic material property information can be derived from the test result.

US 10,955,362 B2 discloses methods for determining the quality of polymers in real-time. Here, a Raman spectrum of a polymer sample is obtained and polymer properties/features are computed by comparing the chemical and/or structural fingerprint to previously stored properties data using a machine learning tool. The algorithms of the machine learning tool are trained with historical Raman data and polymer properties/features of known samples measured in a laboratory with conventional methods. For a desired polymer property, a data set - including the measured polymer properties/features and the respective Raman spectral data is ingested into a database accessible via an application programming interface, API. Here, the structural features are directly measured and a Raman spectrum needs to be recorded. Such measurements and data provision require additional (expensive) equipment and increase the complexity of the method. No intrinsic mechanical property information can be derived from the test result.

As a drawback of all these known concepts, insufficient information is provided. This insufficient information does not enable a determination of intrinsic mechanical properties, such as a temperature- and a strain-rate-dependent stiffness, a yield stress and/or a failure strain.

For instance, a deformation capacity of a material can be different under different loading conditions. Therefore, knowledge of the deformation behavior of the material is an important criterion for material evaluation or material selection. In numerous cases, it has been shown that materials which meet the requirements in a usual strength test, e.g., in a static tensile test, can still fail in practice due to brittle fracture under multiaxial loading and lower temperatures, for example.

Ductility (toughness), stiffness, and brittleness are properties that depend not only on the material, but also on the loading conditions, such as stress state, deformation rate and temperature. Because of these dependencies and the occurrence of multiaxial and/or impact loading in engineering practice, the mechanical response of the material can only be accurately represented by means of temperature- and rate-dependent elastoplastic material models (also referred to as thermo-viscoelastic-viscoplastic material models).

For instance, products, parts and materials used in the automotive industry need to be constant and stable over a large temperature range, e.g. between -30°C to +120°C. However, mechanical properties, in particular of polymers, can be highly dependent and sensitive to temperature changes. For example, poly(methyl methacrylate) is known as a brittle material at a temperature of 4 °C, but its ductile behavior is predominant at higher temperatures.

It has also been considered as being problematic that the known concepts require a tremendous amount of time and computation resources for providing the test results.

So, there is a need for a method that allows to determine material parameters and output material parameters describing at least an elastoplastic mechanical response of one or more materials under impact loading in an automatic manner that is more accurate, less complex and more efficient. Specifically, the output material parameters should describe intrinsic material properties to have a dynamic material response that for instance also covers a strain-rate- and temperature-dependent mechanical response of the material(s).

Summary of the Invention

The above identified problems and needs are solved by the features of the independent claims. Advantageous embodiments can be derived from the dependent claims.

In one aspect, a computer implemented method for outputting parameter values describing at least an elastoplastic mechanical response of one or more materials is disclosed. The method comprises the following steps: inputting, into an optimization algorithm, a set of data values of one or more experimental force-displacement signals derived in a mechanical impact test; searching, by means of the optimization algorithm, a combination of parameter values of a material model representing one or more material properties, wherein this combination of parameter values results in a difference between the set of data values inputted in the inputting step and a prediction of these parameter values, wherein the prediction is based on an estimation that is preferably performed by a trained artificial neural network, wherein the artificial neural network is trained with one or more Finite Element simulations based on the one or more mechanical tests; and outputting, from the optimization algorithm, the combination of parameter values that result in a predefined smallest difference between the set of data values inputted in the inputting step and a finite element simulation or an estimation thereof performed by the artificial neural network.

Thus, an intelligent optimization scheme, preferably a multi-objective optimization algorithm, e.g., as based on genetic algorithms, GA, is performed that searches for the combination of properties that gives the best fit (smallest difference, as predefined) of the inputted set of values to the simulated experimental curve using these best-fit output parameters. This optimization is incorporated into the method to further increase accuracy of the output parameters and to further speed up the method.

This optimization algorithm may be structured as an optimization loop using a comparing step, in which the inputted set of data is compared against an (initial) prediction performed by the artificial intelligence neural network. Afterwards, the optimization algorithm decides whether one or more stopping criteria (e.g., the smallest difference) are reached. If the stopping criterion is not reached, another loop (round) in the optimization algorithm is performed and the comparing and deciding steps are repeated. If the stopping criterion is reached, the optimization algorithm is stopped, and the output parameter values that caused the stopping are used to describe at least an elastoplastic mechanical response of one or more materials that would cause such a set of inputted data values of one or more experimental force-displacement signals derived in a mechanical impact test.

The optimization scheme may utilize external equations during the (parametric) optimization. The optimization scheme (optimization algorithm) may use a NSGAII genetic algorithm, e.g. from a Platypus Python library.

As a result, a thermo-viscoelastic-viscoplastic material model calibrated based on the artificial neural network and a multi-objective optimization scheme is obtained.

In a preferred embodiment, the method further comprises prior to the outputting step the step of predicting the one or more force-displacement signal by using the trained artificial neural network, wherein the artificial neural network comprises one or more neural processing units and mapping, by the trained artificial neural network, material properties representing a constitutive model to the set of data values based on one or more mechanical impact tests.

In a preferred embodiment, the output parameter values describe at least one strain-rate-dependent mechanical response and/or temperature dependent mechanical response. The at least one strain-rate-dependent mechanical response and/or the at least one temperature dependent mechanical response is preferably accounted in an optimization algorithm as described hereinafter.

Although mechanical impact tests are known to be used to extract material response under impact loading, the information obtained directly from these tests does not allow the determination of intrinsic material properties such as the temperature and strain-rate dependent stiffness, yield stress and failure strains. The inventive solution now allows to determine the parameters describing strain-rate- and temperature-dependent mechanical responses of materials under impact loading automatically.

As a result, a thermo-viscoelastic-viscoplastic material model calibrated based on artificial neural networks is obtained.

Although the input data values are obtained from one or more mechanical impact tests, the searching and/or the mapping may be done based on one or more mechanical impact tests. In this regard, the trained artificial neural network may comprise a plurality of neural models, each representing one distinct mechanical impact test. With this mapping step, a real-time computing of finite element (FE) simulations - that are time-consuming and may need adapted parameters in further iterations of the simulations - can be avoided and so, the output parameters are achieved much faster.

Here, a trained artificial neural network is incorporated to replace the usually required FE simulations and thus, computation time to obtain the output parameters is reduced tremendously, e.g. by several orders of magnitude. Here, material properties can be mapped to the input data values, which can comprise one or more mechanical tests. This new setup enlarges the temperature, strain-rate and geometry conditions the method is able to cover, thus increasing its application scope and accuracy.

This method provides parameter values that describe one (or more) elastoplastic mechanical response(s) and can further use additional models accounting for a material's temperature- and strain-rate dependency. In this way, information about the underlying law of physics can be obtained that describe intrinsic material properties and thus, have a higher value for further evaluations and tests based on these output parameters.

This means that this provided solution provides output parameters much faster and more reliable (as result of the trained artificial neural network).

The central concept behind this invention is that the material parameter values that best describe a mechanical impact behavior of one or more material(s) are those which, when used to perform simulations of mechanical impact tests on these materials, produce the smallest difference between such a simulation and the experimental input data. To avoid time-consuming simulations, e.g., when iterations become necessary to achieve a best-fit result, these simulations are now replaced with a trained artificial neural network that is trained from a database of these FE simulations in which one or more mechanical impact tests were used that act as a surrogate model having a computation speed being at least one magnitude higher.

In more detail, this is achieved by inputting experimental data (the set of data values) to the trained artificial neural network, e.g., as part of a computer program product, that has been previously trained to map mechanical properties to corresponding force-displacement signals recorded during one or more impact loading experiments/tests. Output values are constitutive parameters of a material model or, in other words, the mechanical properties of the material.

So, the invention generates material data that can be used to predict the material behavior under conditions outside of those existing in the experiments that were given as input. This information is especially useful in the design of new parts and components by means of computer assisted simulations (e.g., using Finite Element analysis and the like), and to compare different materials in terms of specific values for their mechanical properties.

Using this method can support engineers in the development of new materials, parts and components by providing a tool that allows predicting the material response in specific and arbitrary loading scenarios, more precisely in a mechanical impact loading and for different part geometries, different temperatures, and different strain rates.

In a preferred embodiment, the trained artificial neural network includes one or more feed forward multi-layer-perceptron neural network models, wherein for each mechanical impact test an own neural network model with its own hyperparameters is generated, each model having at least two hidden layers of neurons. In this way, the method becomes most performant and different impact tests can be applied using different neural models. It is possible to run these neural models in parallel and combine the test results to derive the best-fit output parameters.

The multi-layer-perceptron neural network models may be implemented with a Scikit-Learn Python library.

The one or more mechanical test may be any mechanical test conducted at high-velocities or non quasi-static conditions.

The one or more mechanical test may for instance be a high-speed tensile testing or high speed 3-point (or 4-point) bending test; and/or an instrumented Charpy impact test, e.g. according to ISO-Standard 179-2:2020, and/or an instrumented Izod impact test, e.g. according to ASTM Standard D256 or ISO-Standard 180, and/or an instrumented puncture test, e.g. according to ISO-Standard 6603-2:2002-04, and/or an instrumented falling weight test and/or a tensile test, each test preferably performed at different temperatures.

The Charpy Impact test, which can be a notched-impact test also known as the Charpy V-notch test or a unnotched Charpy impact test, is a standardized high strain rate test which determines the amount of energy absorbed by a material during fracture. The absorbed fracture energy is often used as a measure of the material's toughness. It is widely used in industry since it is easy to conduct, and results can be obtained quickly and at comparatively low cost. The apparatus for doing the Charpy impact test consists of a pendulum of known mass and length that is dropped from a known height to impact a probe of material (sample or specimen). The energy transferred to the material can be inferred from the difference in the height of the hammer before and after the fracture (energy absorbed by the fracture event). The probe of material comprises a V-shaped notch. The notch in the sample affects the results of the impact test, thus it is necessary for the notch to be of regular dimensions and geometry. The impact test can be used to measure the toughness of the material. Additionally, the ductile-brittle transition temperature (DBTT) may be derived from the temperature where the energy needed to fracture the material drastically changes.

The Izod impact test, also referred to as Izod impact strength test is another standard method of determining the impact resistance of materials. A pivoting arm is raised to a specific height (constant potential energy) and then released. The arm swings down hitting a notched sample, breaking the specimen. The energy absorbed by the sample is calculated from the height the arm swings to after hitting the sample. A notched sample is generally used to determine impact energy and notch sensitivity. The Izod impact test is similar to the Charpy impact test but uses a different arrangement of the test specimen under test. In the Izod impact test the sample is held in a cantilevered beam configuration whereas it is arranged in a three-point bending configuration in the Charpy impact test.

The instrumented puncture test (IPT) is a category of testing standards that focus on measuring the ability of a sample, usually film, rubber, or paper to withstand being ruptured by a probe when force is applied at a constant speed.

The instrumented falling test or drop test (*e.g.* a falling weight or dart drop impact test, DDI) is a mechanical test of components in which the component is dropped from a defined height onto a defined surface.

In a preferred embodiment, the artificial neural network is trained with data from a database of a plurality of finite element (FE) simulations. In that database, there may be a plurality of data entries, each data entry may represent one test result of one simulation of one (or more) mechanical test(s) with a fixed parameterization. This data entry is used to map one or more mechanical properties to a corresponding force-displacement signal.

The set of input data values may be a set of numerical data in the form of at least one multi-point force-displacement signal or a series of data values derived thereof.

The set of input data values for the optimization algorithm may represent elasticity data. An elasticity data has an information about the ability of a material to resist a distorting influence and to return to its original size and shape when that influence or force is removed. Solid objects will deform when adequate loads are applied to them; if the material is elastic, the object will return to its initial shape and size after removal. Elasticity and the elastic modulus are defined as a force per unit area, generally a measurement of pressure, which in mechanics corresponds to stress.

The set of input data values for the optimization algorithm may represent a plasticity data. A plasticity data has an information about the ability of a material to undergo permanent deformation, a non-reversible change of shape in response to applied forces. It is, thus, the opposite of elasticity.

The transition from an elastic behavior of a material to a plastic behavior of that material is known as yielding. The set of input data values may represent a (rate-dependent) yield information. The yield information may be information about a yield point that is the point on a stress-strain curve that indicates the limit of elastic behavior and the beginning of plastic behavior of a material. Below the yield point, a material will deform elastically and will return to its original shape when the applied stress is removed. Once the yield point is passed, some fraction of the deformation will be permanent and non-reversible and is known as plastic deformation.

A rate-dependent yield information is an information to define a material's yield behavior accurately when a yield strength depends on the rate of straining and the anticipated strain rates are significant. This rate-dependent yield information can be conveniently defined on the basis of work hardening parameters and field variables by providing tabular data for isotropic hardening metal plasticity models, isotropic component of nonlinear isotropic/kinematic plasticity models, and extended Drucker-Prager plasticity model. This rate-dependent yield information can be defined through specification of user-defined overstress power law parameters, yield stress ratios, or Johnson-Cook rate dependence parameters. This rate-dependent yield information should be specified such that the yield stress increases with increasing strain rate, especially in a dynamic analysis.

The set of input data values for the optimization algorithm may represent a damage and/or a failure information. This is an information about a damage evolution capability for ductile materials. The damage may be a progressive degradation of the material stiffness, leading to material failure. The damage and/or failure information may be based on mesh-independent measures, either plastic displacement or physical energy dissipation, to indicate the evolution of a damage after a damage initiation. This information may take into account the combined effect of different damage mechanisms acting simultaneously on the same material and may include options to specify how each mechanism contributes to the overall material degradation.

Prior to the inputting step, the method may comprise a featurization step to reduce the number of input data values for the optimization algorithm. The definition and determination of such features is dependent on whether the features are present in different experimental impact test results and whether they are useful as (target) output parameter values for reducing computational effort.

In either case, since the experimental data are multi-point curves, it is advantageous to reduce this information to a smaller set of input data values which contain the most important information in a condensed form. This allows for more compact artificial neural models, it accelerates a model calibration, it dismisses artifacts, and it allows for parameter weight regularization.

Preferably, a limit load, e.g. referenced as $F_{max}$, as one or more of the parameters represents the input data values. The limit load is the maximum load that a material (a structure) can safely carry. In other words, it is the load at which the structure is in a state of incipient plastic collapse. As the load on the structure increases, the displacements increase linearly in the elastic range until the load attains the yield value. Beyond this, the load-displacement response becomes non-linear, and the plastic or irreversible part of the displacement increases steadily with the applied load. Plastic deformation spreads throughout the solid and at the limit load, the plastic zone becomes very large, the displacements become unbounded and the component is said to have collapsed.

Further preferably a deflection, e.g. referenced as $U_{max}$; being the deflection at the limit load, as one or more of the parameters represents the input data values. The deflection is the degree to which a part of a structural element is displaced under a load, e.g. the limit load, because it deforms. It may refer to an angle or a distance. The deflection distance of a member under a load can be calculated by integrating the function that mathematically describes the slope of the deflected shape of the member under that load. More preferably, the deflection is directly calculated from a sensor measurement of the one or more experimental force-displacement signals.

It is also preferred when a strain energy, in particular the area under the force-displacement signals up to a 5% drop from a peak load, e.g. referenced as $E_{br}$, as one or more of the parameters represents the input data values.

The aforementioned parameters are particularly preferred as input data values when the one or more mechanical test is an instrumented Charpy impact test or an instrumented puncture test.

If the one or more mechanical test is a quasi-static tensile test, a slope, e.g. referenced as E of the stress curve in a predefined strain range, in particular a slope corresponding to the least squares regression between 0.05% and 0.25% strain, may represent the input data values as one or more of the parameters.

In the latter case, a yield strength may further as one or more of the parameters represent the input data values. A yield strength is a value that refers to stress at the yield point, preferably being an initial yield stress at zero offset strain. The yield point is the point on a stress-strain curve that indicates the limit of elastic behavior and the beginning of plastic deformation. Below the yield point, a material will act elastically and will return to its original shape when the applied stress is removed. Once the yield point is passed, some fraction of the deformation will be permanent and non-reversible and is known as plastic deformation.

The material properties as output parameter may refer to a thermo-viscoelastic-viscoplastic model. Viscoplasticity describes the rate-dependent inelastic property of materials. In addition, viscoelasticity is the property of materials that exhibit both viscous and elastic characteristics when undergoing deformation. The output parameters of this model describe both material properties in dependence to the temperature so as to have a model that describes intrinsic material properties dependent on temperature and strain-rate.

The outputted parameter values may be one or more of: a geometry dependent (meaning a specific state of stress/strain multiaxiality), a temperature dependent and a strain-rate dependent stiffness, yield stress and failure strain.

The outputted parameter values may include at least one intrinsic material property, and the outputted parameter values may describe features suitable for predicting the material property in conditions outside of the set of input data values. In this way, the method becomes a useful tool for material property prediction for different kinds of industries, for instance the automotive industry, in which environmental parameters may vary over a large range.

The constitutive model for the material may be based on a non-linear model for elasticity or plasticity and preferably there is a failure parameter calibration (meaning it accounts for failure behavior).

The constitutive model for the material may be based on a non-linear model for elasticity. Materials have elastic behavior usually only up to a certain load. After that, the deformations are plastic. Materials may have a non-linear elastic stress-strain curve, - in such cases non-linear elastic models best describe the material. Such models are, for instance, Blatz-Ko Rubber, Mooney-Rivlin Rubber and viscoelastic models. In the area of inelasticity, the different materials react differently.

The constitutive model for the material may, additionally or in the alternative, be based on a non-linear model for plasticity. In a model undergoing inelastic deformation, both normal and shear components of stress undergo change under combined stress fields, corresponding to the increment of either normal or shear component of strain.

The constitutive model may be one or more of the models as described in ABAQUS 2016 Analysis User's Guide, Part V: Materials. Dassault Systemes Simulia, Inc.

The constitutive model may be one or more of the models as described in LS-DYNA, KEYWORD USE''S MANUAL, VOLUME II-Material Models, 2021, Livermore Software Technology Corporation.

The constitutive model may be one or more of the following models: a linear elasticity model, based on Hooke's law; a linear viscoelasticity model; a hyperelasticity model (e.g. Arruda-Boyce, Ogden, Marlow, Mooney-Rivlin, Neo-Hookean, Polynomia); a linear elastic isotropic strain hardening plasticity model (Mises or Hill yield surfaces with associated plastic flow, which allow for isotropic and anisotropic yield, respectively); an ABAQUS models, such as an ABAQUS perfect plasticity model or an ABAQUS isotropic hardening model or an ABAQUS kinematic hardening model or an ABAQUS direct entry of test data model (e.g. an ABAQUS direct entry of test data model or an ABAQUS Two-layer viscoplasticity model or an ABAQUS yield stress ratios model or an ABAQUS progressive damage and failure for ductile metals model or an ABAQUS progressive damage and failure for fiber-reinforced materials model; a Johnson-Cook isotropic hardening model; a Drucker-Prager model; a Mohr-Coulomb plasticity model; a crushable foam plasticity model; a Ramberg-Osgood plasticity model; a LS-DYNA MAT_24 model; a Johnson-Cook rate dependence model; a Bergstrom-Boyce model; a Bergstrom Three Network model; a Bergstrom Hybrid model; a LS-DYNA SAMP-1 model; and/or a LS-DYNA model such as MAT_81, MAT_105, MAT_107 or MAT_120 isochoric damage models (Lemaitre) non-isochoric damage models for metals (Gurson) non-isochoric damage models for plastics (SAMP), modeling of crazing).

In a preferred embodiment, the one or more experimental force-displacement signal was recorded during an impact loading experiment.

According to another aspect, a non-transitory computer readable storage medium for tangibly storing computer program instructions capable of being executed by a processor is provided, the computer program instructions defining the steps of the method as described above.

Brief Description of the Drawings

Embodiments of the invention are illustrated by way of example and are not limited by the figures of the accompanying drawings in which identical references represent similar elements.

Fig. 1 shows an exemplary flow diagram illustrating method steps of a computer-implemented method according to the invention;

Fig. 2 shows an exemplary structure of an artificial neural network used for performing method steps of a computer-implemented method according to the invention;

Fig. 3a shows an exemplary set of input data according to the invention;

Fig. 3b shows an exemplary set of output parameters according to the invention;

Fig. 4 shows an exemplary optimization scheme;

Figs. 5a to 5c illustrate respective experimental IPT, INC and tensile curves at different temperatures from -30°C to 50°C for polymer material A;

Figs. 6a to 6c illustrate respective experimental IPT, INC and tensile curves at different temperatures from -30°C to 50°C for polymer material B;

Figs. 7a to 7d illustrate output parameters representing material properties in graphical form for material A; and

Figs. 8a to 8d illustrate output parameters representing material properties in graphical form for material B.

Detailed Description of the Drawings

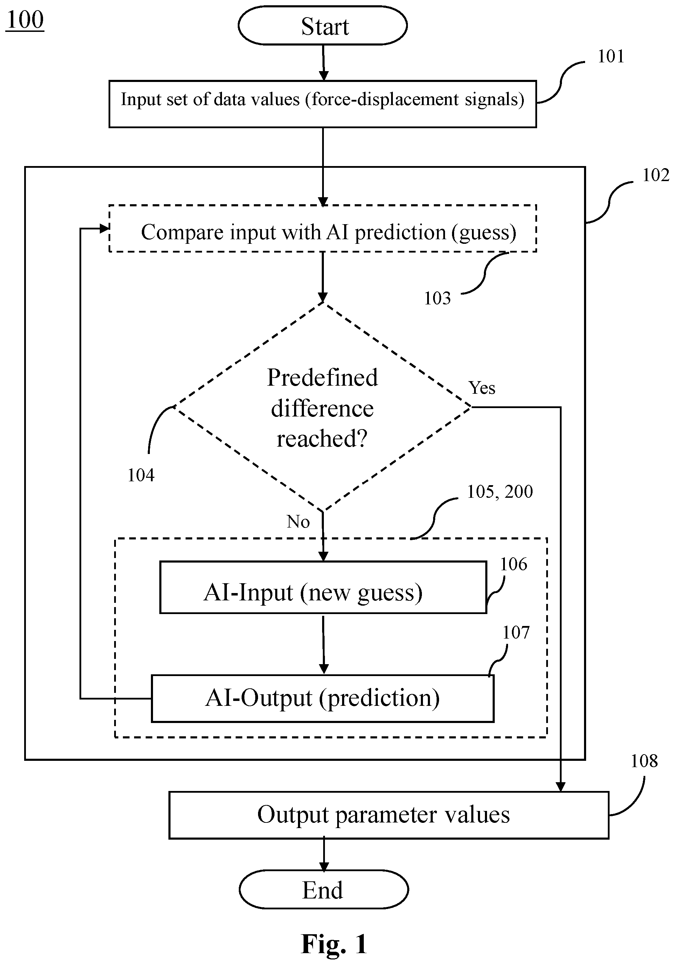

Fig. 1 shows an exemplary flow diagram illustrating method steps of a computer-implemented method 100 according to the invention. Optional steps of the method 100 are presented in dashed lines.

The method is for outputting parameter values describing at least an elastoplastic mechanical response of one or more materials.

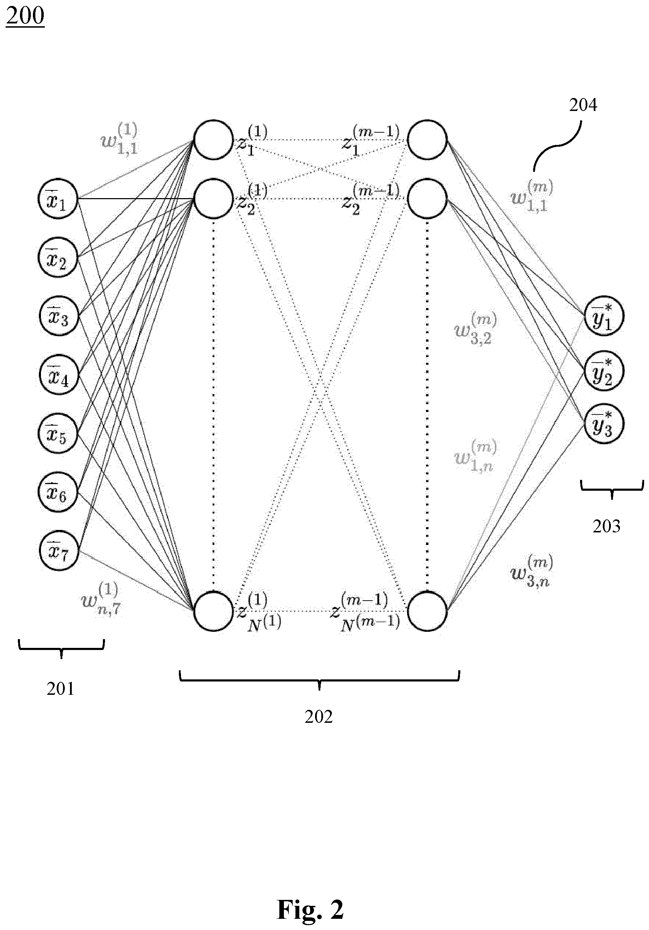

In step 101, an inputting step is shown. In step 101, an inputting step is shown. Here, a set of data values of one or more experimental force-displacement signals derived in a mechanical impact test is input into an optimization algorithm that further comprises a trained artificial neural network, ANN, 200 (see Fig. 2) comprising one or more neural processing units.

In an optional step (not shown in Fig. 1), a featurization step is shown in which several input data values that were input in step 101 are further reduced.

In step 102, the optimization algorithm (optimization scheme) is shown. In this optimization algorithm, a combination of parameter values is searched which provide a smallest possible (best fit) difference between the input parameters (step 101) and a prediction of these parameter values performed by the artificial neural network 200.

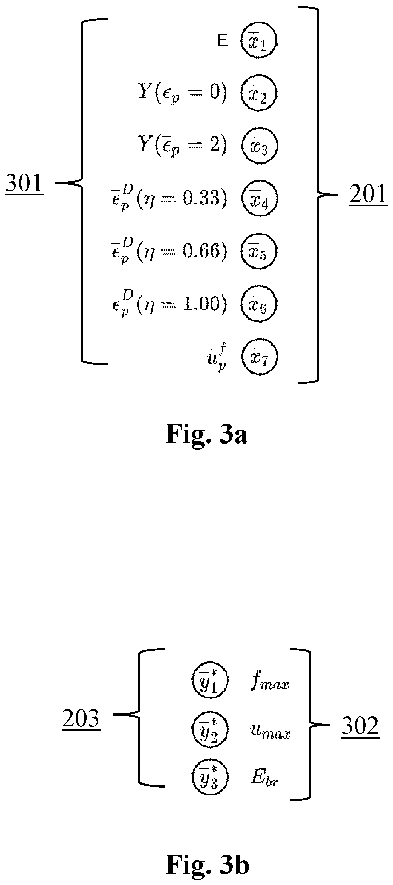

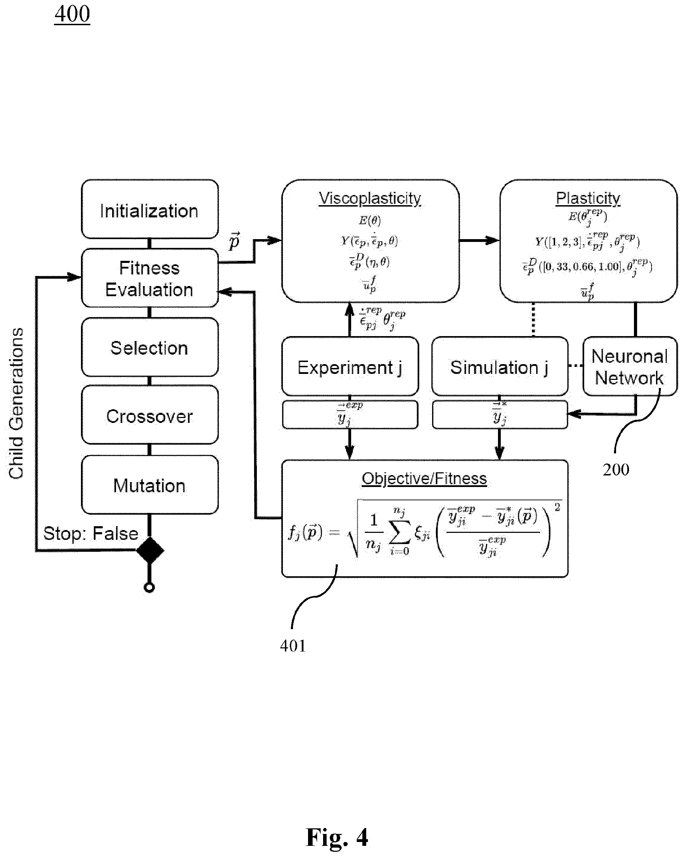

In step 108, an outputting step is shown, in which the combination of parameter values 302 (as for instance listed in Fig. 3b) that result in a predefined smallest difference between the set of input data values based on experimental force-displacement signals 101 and a prediction of these signals derived from the ANN 200 performed using guessed combination(s) of parameter values of step 106 are outputted by the optimization algorithm 102. If multiple experimental signals are used as input (e.g. INC, IPT and TEN and also different test temperatures), then the optimization loop produces simultaneously optimal parameter sets 301/201 each corresponding to the specific strain-rate and temperature of the corresponding input signal (as shown in Fig.4). These sets are constrained by equations 1 and 2 which result in the optimization algorithm producing the parameter set shown in table 4. Thus, the optimization can determine the temperature-viscoelastic-viscoplastic material properties.

The optimization algorithm comprises a comparing step 103. Here, the set of input data values (step 101) is compared with predicted signals derived from the ANN 200. Initially, these predicted signals by the ANN 200 are generated using an initial guess of the parameters 301/201.

Following to the comparing step 103 is a decision step 104 in which the comparison result (providing a difference between input and prediction) is compared with one or more stopping criteria, e.g. a smallest difference (best fit) criterion. If it is decided in step 104 that the stopping criteria is/are met (yes-case), the optimization algorithm is ended and the obtained parameter values are output in step 108.

If it is decided in step 104 that the stopping criteria is/are not met (no-case), the optimization algorithm is continued. Then, the ANN 200 obtains new guesses for the input values 301 in step 106 (these guesses are generated automatically by the optimization algorithm) and predicts the corresponding force-displacement signal from these input data values 301 in step 107. This step 106 may also be referred to "local input" of the AI model performed by the optimization algorithm. The AI model then predicts a force-displacement signal as a "local output" in step 107. This prediction is produced based on the ANN 200 which was trained with Finite Element simulations in advance. The output of the step 107 (the prediction) is then provided back to the comparing step 103.

This optimization algorithm utilizes the prediction from step 107 and does calculate the output parameters as long as the stopping criteria in step 104 are not met, so the optimization algorithm is an optimization loop, preferably a multi-objective optimization algorithm, most preferably a genetic multi-objective optimization algorithm, for iteratively searching a combination of material properties that best-fit to the input data set that represents one or more force-displacement signals derived in a mechanical impact test. In this optimization algorithm 102, having searching steps 103 and 104, a combination of parameter values of the material model that result in the smallest difference between the inputted set of data values and a simulation obtained by using the guessed values of the parameter values of the material model (so-called "best-fit") are searched by means of an optimization algorithm.

For implementation of the invention, a computer program product may comprise the following three key elements, (1) a neural-network-based model, NNM that comprises one or more neural processing units and predicts essential features of an impact test given an arbitrary set of material parameters; (2) a neural-network parameter set, NNS, which stores learned by the NNM. The NNS is trained using a database that was generated by performing a large number of calculations, e.g. via Finite Element simulations; and (3) a multi-optimization optimization scheme that uses the NNM to find the set of material parameters that best fit a given experiment according to a selected criterion.

The input set of data values 101 for the optimization loop (step 102) are based on experimental from which material properties should be determined.

Experimental supplementary data most promising as a basis for this particular invention are, for example, obtained from a quasi-static tensile test (TEN) at two different temperatures, namely - 30°C and +23°C.

Experimental data most promising as a basis for this particular invention are, for example, obtained from an instrumented impact puncture test, hereinafter short IPT.

Experimental data most promising as a basis for this particular invention are, for example, obtained from an instrumented Charpy impact test, herein short INC. This Charpy impact test can in principle be a V-notched Charpy impact test or an unnotched Charpy impact test.

These tests (TEN, INC, IPT) produce numerical data in the form of a force-displacement signal (curve). Within the method 100, the combination of material properties which best-fit these experimental results from the tests TEN, INC, IPT can be found with reduced computation and time effort.

Since the experimental data are represented as multi-point force-displacement signals (curves), it is advantageous to reduce the set of input values of step 101 to a smaller set of input values which contain the most important information in condensed-form. This allows for more compact artificial intelligence models, accelerates model calibration, dismisses artifacts, and allows for parameter weight regularization. This featurization step requires domain knowledge and an understanding of the overall test system.

Exemplarily, for IPT and INC defined features are:

- limit load $F_{max}$
- deflection $U_{max}$ (at $F_{max}$)
- area under the force-displacement signals up to a 5% force drop of the peak load $E_{br}$.

Exemplarily, for TEN defined features are:

- slope E corresponding to the least squares regression between 0.05% and 0.25% strain
- yield stress $\sigma_y$.

The objective of the constitutive material model is to describe the mechanical behavior of a material. For the example as illustrated with the Figures, a linear elastic isotropic strain hardening Mises plasticity model has been used, the input parameters of which can be input in tabular form in ABAQUS. The input parameters are the set of input data 201 for the ANN 200. As indicated above, other constitutive material models to describe the material mechanical behavior can also be used.

The particular material model as illustrated with the Figures is neither strain-rate dependent nor temperature dependent. However, since there are different strain-rates in each of the three experimental data that are considered (TEN, INC, IPT) and each of the tests that provides the experimental data was performed at different temperatures, it is necessary to have a strain-rate dependent and/or temperature dependent material model.

In this example, non-linear models for elasticity

$$E(\theta) = A^E \left( 1 + C^E ln \left( \frac{\dot{\epsilon}_0^{dyn}}{\dot{\epsilon}_0^{qs}} \right) \right) \left( 1 - T^{*m^E} \right) \tag{1}$$

and for plasticity

$$Y(\bar{\epsilon}_p, \dot{\bar{\epsilon}}_p, \theta) =$$
$$\left( A^Y + B^Y \bar{\epsilon}_p{}^{n^Y} \right) \left( 1 + C^Y ln \left( \frac{\dot{\bar{\epsilon}}_p}{\dot{\epsilon}_0^{qs}} \right) \right) \left( 1 - T^{*m^Y} \right) \tag{2}$$

are implemented into the optimization loop as shown in 400.

Equation (1) represents an additional model for strain-rate and temperature dependent elasticity whereas equation (2) represents an additional model for strain-rate and temperature dependent plasticity.

The implemented material model as illustrated with the Figures also allows the calibration of failure parameters by implementing a ductile damage model dependent of temperature and stress-triaxiality:

$$\bar{\epsilon}_p^D(\eta, \theta) = \begin{cases} \frac{L^{tri}}{1 + e^{-k^{tri}(\theta - \theta_t^{tri})}} & \eta = 1.00 \\ \frac{L^{bi}}{1 + e^{-k^{bi}(\theta - \theta_t^{bi})}} & \eta = 0.66 \\ \bar{\epsilon}_p^D(0.66, \theta) * R & \eta = 0.33 \\ 0.35 & \eta = 0.00 \\ 100 & \eta = -0.33 \end{cases} \tag{3}$$

Where $\bar{\epsilon}_p^D$ is the equivalent plastic strain at the onset of ductile damage. The complete definition of the implemented material model as illustrated with the Figures amounts to a total of five temperature- and strain-rate-dependency parameters and six failure parameters to be determined, see following table 1:

*Table 1 - parameters for temperature- & strain-rate dependency & failure*

| Param. | Unit | Description | Boundaries |
|---|---|---|---|
| $\rho$ | $\frac{ton}{mm^3}$ | Density | $9e^{-10}$ |
| $\nu$ | - | Poisson's Ratio | 0.40 |
| $\dot{\epsilon}_0^{qs}$ | $s^{-1}$ | Quasistatic rate | 0.01 |
| $\dot{\epsilon}_0^{dyn}$ | $s^{-1}$ | Dynamic rate | 254 |
| $\theta_0$ | $°C$ | Reference Temperature | -30 |
| $0_m$ | $°C$ | Melt Temperature | [140,180] |
| $A^E$ | $MPa$ | Reference Stiffness | 2840 |

(continued)

| Param. | Unit | Description | Boundaries |
|---|---|---|---|
| $C^E$ | - | Strain rate coefficient | [0.02, 0.10] |
| $m^E$ | - | Temperature exponent | [0.20, 0.80] |
| $A^Y$ | MPa | Reference yield strength | 42 |
| $B^Y$ | - | Strain hardening coeff. | 5 |
| $C^Y$ | - | Strain rate coefficient | [0.02, 0.06] |
| $m^Y$ | - | Temperature exponent | [0.20, 0.60] |
| $n^Y$ | - | Strain hardening exponent | 1 |
| $L^{tri}$ | - | Max. plastic strain at fail. | [0.50, 0.75] |
| $k^{tri}$ | $°C^{-1}$ | Logistic growth rate | [0.05, 0.18] |
| $\theta_t^{tri}$ | $°C$ | Ductile-brittle-transition | [30, 50] |
| $L^{bi}$ | - | Max plastic strain at fail. | [1.20, 3.50] |
| $k^{bi}$ | $°C^{-1}$ | Logistic growth rate | [0.05, 0.18] |
| $\theta_t^{bi}$ | $°C$ | Ductile-brittle-transition | [-20, 0] |
| $R$ | - | Uniaxial to biaxial ratio | 0.85 |
| $\overline{u}_p^f$ | - | Displacement at fail. | 0.01 |

The column "boundaries" in this table 1 reflects the boundaries of each individual parameter. Parameters in which only a single value is listed in this column are fixed and thus no boundaries for this parameter needs to be determined.

As stated above, computed FE simulations are used to generate (to form) a database to train the ANN 200. The ANN 200 then replaces (surrogates) these FE simulations by delivering a much faster response in the mapping step 107.

As mentioned above, the FE simulations only account for the elasto-plastic response, whereas the strain-rate dependency and the temperature dependency and the calibration of failure parameters is accounted for with equations (1) to (3) e.g. during optimization step 104. A collection of 36.878 simulations for INC and 22.755 simulations for IPT, respectively, has been run. Each simulation involved a different parameter combination based on the values shown in following table 2:

*Table 2 - Elasto-plastic material parameter used to create FE simulation database used during mapping step 103*

| Parameter | Unit | Database Values |
|---|---|---|
| E | MPa | [250, 500, ... 4000, 4500] |
| $Y(\overline{\varepsilon}_p = 0)$ | MPa | [15, 20, 25, ... 50, 55, 60] |
| $Y(\overline{\varepsilon}_p = 1)$ | MPa | [5] + $Y(\overline{\varepsilon}_p = 0)$ |
| $Y(\overline{\varepsilon}_p = 2)$ | MPa | [-10, 5, 40] + $Y(\overline{\varepsilon}_p = 1)$ + $Y(\overline{\varepsilon}_p = 0)$ |
| $\overline{\epsilon}_p^D(\eta = 0.33)$ | - | [0.05, 0.1, 0.25, ... 2.5, 3.0, 3.5] |
| $\overline{\epsilon}_p^D(\eta = 0.66)$ | - | [0.05, 0.1, 0.25, ... 2.5, 3.0, 3.5] |
| $\overline{\epsilon}_p^D(\eta = 1.00)$ | - | [0.05, 0.1, 0.25, ... 2.5, 3.0, 3.5] |
| $\overline{u}_p^f$ | mm | [0, 0.05, 0.1] |

Fig. 2 shows an exemplary structure of an artificial neural network 200 used within the method 100, especially for the mapping step 103 as shown in Fig. 1.

An Artificial Neural Network, ANN uses a network of neurons 201, 202, 203 to process the set of input values inputted into the ANN 200 and to generate output parameters 302 from the ANN 200. For example, each neuron 201, 202, 203 in the ANN 200 receives a set of input data 301 in step 102 of Fig. 1. Some of the input data value 301 to a neuron may be the outputs of certain other neurons in the ANN 200. Some of the inputs to a neuron may be the inputs provided to the ANN 200. The input/output relations among the neurons 201, 202, 203 in the ANN 200 represent the neuron connectivity in the ANN 200.

For example, each neuron 201, 202, 203 can have a bias, an activation function, and a set of synaptic weights 204 for its inputs respectively. The activation function may be in the form of a step function, a linear function, a log-sigmoid function, etc. Different neurons 201, 202, 203 in the ANN 200 may have different activation functions.

For example, each neuron 201, 202, 203 can generate a weighted sum of its inputs and its bias and then produce an output that is the function of the weighted sum, computed using the activation function of the neuron 201, 202, 203.

The relations between the input(s) and the output(s) of the ANN 200 in general are defined by an ANN model that includes the data representing the connectivity of the neurons 201, 202, 203 in the ANN 200, as well as the bias, activation function, and synaptic weights of each neuron 201, 202, 203. Based on a given model, a computing device can be configured to compute the output(s) of the ANN 200 from a given set of inputs to the ANN 200.

In general, an ANN 200 may be trained using a supervised method where the parameters in the ANN 200 are adjusted to minimize or reduce the error between known outputs associated with or resulted from respective inputs and computed outputs generated via applying the inputs to the ANN 200. Examples of supervised learning/training methods include reinforcement learning and learning with error correction.

Alternatively, or in combination, an ANN 200 may be trained using an unsupervised method where the exact outputs resulted from a given set of inputs is not known before the completion of the training. The ANN 200 can be trained to classify an item into a plurality of categories, or data points into clusters.

Multiple training algorithms can be employed for a sophisticated machine learning/training paradigm.

Deep learning uses multiple layers of machine learning to progressively extract features from input data. For example, lower layers can be configured to identify edges in an image; and higher layers can be configured to identify, based on the edges detected using the lower layers, items captured in the image, such as faces, objects, events, etc. Deep learning can be implemented via ANN 200, such as deep neural networks, deep belief networks, recurrent neural networks, and/or convolutional neural networks.

At least some embodiments disclosed herein provide a general-purpose integrated circuit device configured to perform computations of ANN 200. An integrated circuit device may include a Deep Learning Accelerator, DLA, and a random access memory, RAM and/or a direct media access, DMA, controller. The integrated circuit device can be configured with separate connections for concurrent access to the RAM

The DLA may include a set of general-purpose, programmable hardware computing logic that is specialized and/or optimized to perform parallel vector and/or matrix calculations, including but not limited to multiplication and accumulation of vectors and/or matrices. Further, the DLA can include one or more Arithmetic-Logic Units, ALUs, to perform arithmetic and bitwise operations on integer binary numbers.

The DLA is programmable via a set of instructions to perform the computations of the ANN 200.

The granularity of the DLA operating on vectors and matrices corresponds to the largest unit of vectors/matrices that can be operated upon during the execution of one instruction by the DLA. During the execution of the instruction for a predefined operation on vector/matrix operands, elements of vector/matrix operands can be operated upon by the DLA in parallel to reduce execution time and/or energy consumption associated with memory/data access. The operations on vector/matrix operands of the granularity of the DLA can be used as building blocks to implement computations on vectors/matrices of larger sizes.

The implementation of a typical/practical ANN 200 involves vector/matrix operands having sizes that are larger than the operation granularity of the DLA. To implement such ANN 200 using DLA, computations involving the vector/matrix operands of large sizes can be broken down to the computations of vector/matrix operands of the granularity of the DLA. The DLA can be programmed via instructions to carry out the computations involving large vector/matrix operands. For example, atomic computation capabilities of the DLA in manipulating vectors and matrices of the granularity of the DLA in response to instructions can be programmed to implement computations in an ANN 200.

A typical ANN 200 can be described/specified in a standard format (e.g., Open Neural Network Exchange (ONNX)). A compiler can be used to convert the description of the ANN 200 into a set of instructions for the DLA to perform calculations of the ANN 200. The compiler can optimize the set of instructions to improve the performance of the DLA in implementing the ANN 200.

For example, a stream of input data 301 to the ANN 200 can be configured in the form of a sequence of input data sets. Each input data set is for a set of input to the ANN during a time slot. While the DLA is computing the output from the current set of input, the DMA controller can store the next set of input into the RAM; and the CPU can concurrently retrieve, from the RAM, the output generated for the previous set of input. Thus, the task of preparation and processing of input data to the ANN 200 can be offloaded from the CPU. The combination of the DLA, RAM and DMA controller can function as an independent supplier of results from ANN 200 to the CPU. The CPU can retrieve a set of output at a time when the output is needed. The CPU can instruct the DMA controller to pause its operations in supplying input to the ANN when output from the ANN 200 is not required. Subsequently, when output from the ANN 200 is needed, the CPU can instruct the DMA controller to resume its operations of loading input data into the RAM

To model the IPT and INC tests for the ANN 200 as shown in Fig. 2 to perform the mapping step 107 of Fig. 1, a feed forward multi-layer-perceptron neural network models may be implemented, here preferably with a Scikit-Learn Python library. Each test (IPT, INC) generates its own ANN model with its own hyperparameters.

The network input neurons 201 as shown in Fig. 2 are fed with the material parameters as defined table 2. The preferred set of input data for predicting material properties based on IPT and INC tests are listed in Fig. 3a.

The network outputs (predicts) with neurons 203 the above listed features for TEN, IPT, INC as output parameters 302 as follows:

For the IPT and INC tests, the output parameters 302 are (see also Fig. 3b):

- limit load $F_{max}$
- deflection $U_{max}$ (at $F_{max}$)
- area under the force-displacement signals up to a 5% force drop of the peak load $E_{br}$.

The ANN 200 as shown in Fig. 2 has two hidden layers with $N^{(1)}$ and $N^{(2)}$ neurons 202. For each layer a rectified linear unit activation may be used to introduce non-linearity.

For the ANN 200 training of the INC and IPT, an optimization algorithm can be employed, e.g. a direct grid search approach to determine the model hyperparameters as listed in following table 3:

*Table 3 - AI model hyperparameter*

|  | min | max | △ | INC | IPT |
|---|---|---|---|---|---|
| $N^{(1)}$ | 100 | 500 | 100 | 300 | 300 |
| $N (2)$ | 100 | 500 | 100 | 400 | 300 |
| log $\alpha$ | -6 | -2 | 1 | -5 | -5 |
| $b$ | 200 | 1.000 | 200 | 200 | 400 |

To calibrate the material model - meaning to determine the material properties that best fit the experimental results of the input data 301-- a multi-objective optimization algorithm is applied as shown in Fig. 1 and in greater details shown in Fig. 4.

In this way, when the algorithm searches for the best solution (=predefined smallest difference between the set of data values 301 inputted in the inputting step 102 and a finite element simulation performed with this combination of parameter values 302), it must do it for all mechanical impact tests (such as INC, IPT, tensile tests) at all temperatures simultaneously while also satisfying the additional constraints imposed by the strain-rate and temperature dependencies described with equations (1) to (3).

In Fig. 4, a framework is illustrated that allows for an arbitrary number of experiments, namely mechanical impact tests (for instance INC, IPT, TEN) in a grid of representative strain-rates and temperatures. Combined constitutive output parameters 302 can be found that are defined by equations (1), (2) and (3). The entire scheme produces a table of material properties, see table 4:

*Table 4 - Material properties determined with the invention*

| $\theta_m$ | $C^E$ | $m^E$ | $C^Y$ | $m^Y$ |  |
|---|---|---|---|---|---|
| 152 | 0.07 | 0.30 | 0.051 | 0.54 |  |

| $k^{bi}$ | $\theta_t^{bi}$ | $L^{bi}$ | $k^{tri}$ | $\theta_t^{tri}$ | $L^{tri}$ |
|---|---|---|---|---|---|
| 0.077 | -16 | 3.48 | 0.09 | 30 | 0.78 |

These material properties of table 4 are calibrated for different temperatures and strain-rates. This collection of output parameters 302 according to table 4 are thermo-viscoelastic-viscoplastic model parameters.

The complete parameter determination scheme within the optimization 400 by using ANN 200 is illustrated in Fig. 4. A feedback loop provides parameters for elasticity and plasticity to the ANN 200 and the output values of the ANN 200 are compared with an objective/fitness unit 401.

The generalization performance of the ANN 200 is listed in table 5. This is quantified by means of the coefficient of determination (R2) and the mean absolute error (MAE).

*Table 5-- Generalization performance for ANN 200*

|  | $^{IPT}R2$ | $^{IPT}MAE$ | $^{INC}R2$ | $^{INC}MAE$ |
|---|---|---|---|---|
| $f_{max}$ / N | 0.997 | 21.29 | 0.988 | 3.91 |
| $u_{max}$ / mm | 0.970 | 0.22 | 0.976 | 0.12 |
| $E_{br}$ / J | 0.996 | 0.28 | 0.992 | 0.017 |

To run the optimization scheme 400, six experiments were used for calibration, namely an IPT experiment at 23°C, an IPT experiment at -30°C, an INC experiment at 23°C, an INC experiment at -30°C, a TEN experiment at -30°C and a TEN experiment at 30°C. The remaining IPT and INC experiments at temperatures of 0°C, 30°C and 50°C were used merely for validation in order to evaluate how accurate the ANN 200 predicts these experiment curves. The optimization scheme 400 was configured to run with 100 generations each having a population of 200 individuals representing sets within the bounds of table 1.

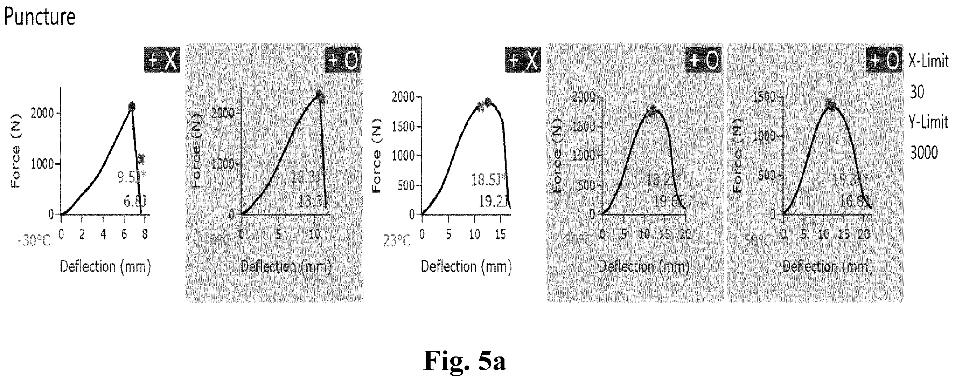

Figs. 5a to 5c illustrate respective experimental IPT, INC and tensile curves at two different temperatures for polymer material A. Material A is a composition of 59.7 wt.-% Polypropylene post-consumer recyclate (commercially available by Borealis AG, with a density according to ISO 1183 of 915 kg/m$^3$, an MFR$_2$ at 230 °C according to ISO1133 of 20.0 g/10 min) + 40 wt.-% HECO1 (heterophasic polypropylene commercially available by Borealis AG, with an MFR$_2$ at 230 °C according to ISO1133 of 11.0 g/10 min and intrinsic viscosity of 2.1 dl/g) + 0.3 wt.-% Irganox B225 (antioxidant, commercially available by BASF). When referring to Material A hereinafter, this composition is meant.

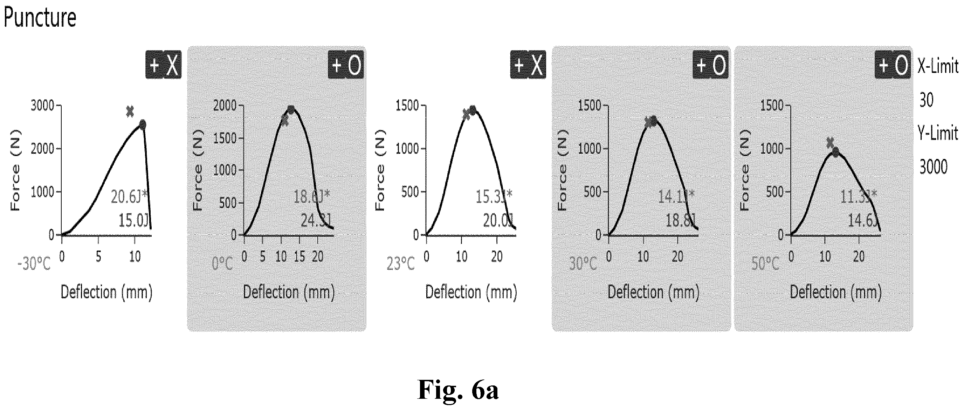

Figs. 6a to 6c illustrate respective experimental IPT, INC and tensile curves at two different temperatures for polymer material B. Material B is a composition of 79.7 wt.-% Post-consumer recyclate polyblend PE/PP (commercially available by Borealis AG, with a density according to ISO 1183 of 940 kg/m$^3$, an MFR$_2$ at 230 °C according to ISO1133 of 5.5 g/10 min) + 20 wt.-% Queo8201 (ethylene based 1-octene plastomer, commercially available by Borealis AG, with a density according to ISO 1183 of 883 kg/m$^3$, an MFR$_2$ at 190 °C according to ISO1133 of 1.1 g/10 min) + 0.3 wt.-% Irganox B225 (antioxidant, commercially available by BASF). When referring to Material B hereinafter, this composition is meant.

For materials A and B the respective experimental IPT curve is shown in Figs. 5a and 6a at different temperatures from -30°C to 50°C.

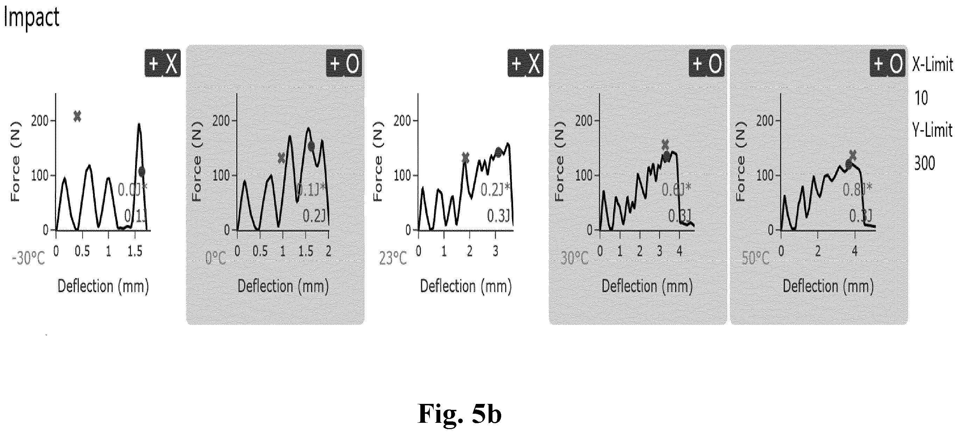

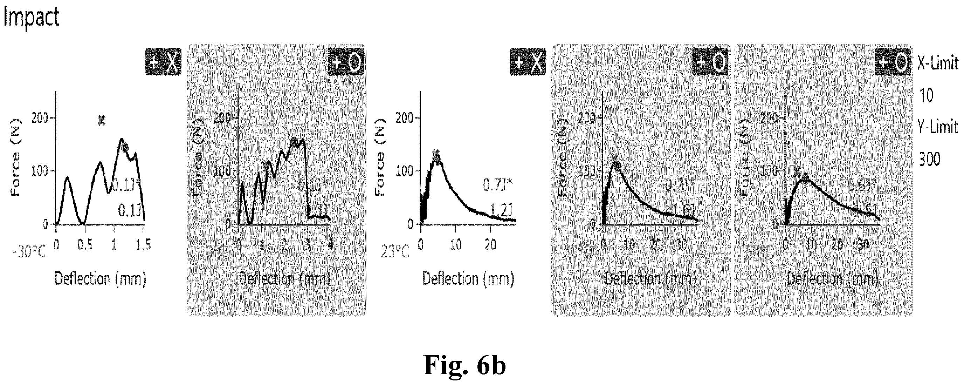

For materials A and B the respective experimental INC curve is shown in Figs. 5b and 6b at different temperatures from -30°C to 50°C.

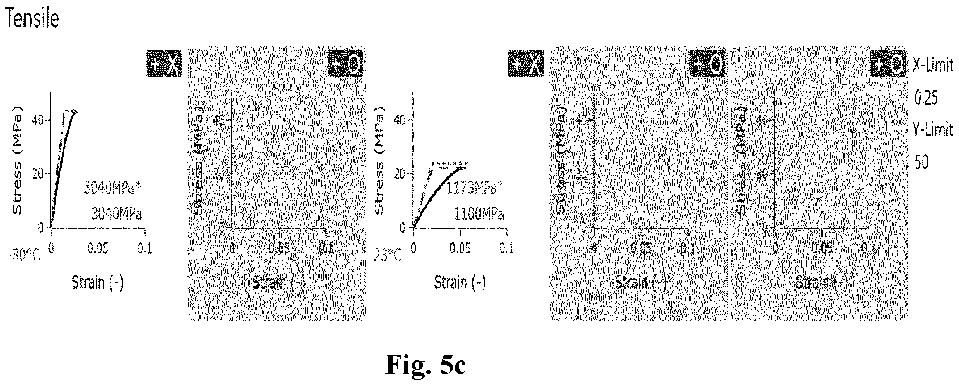

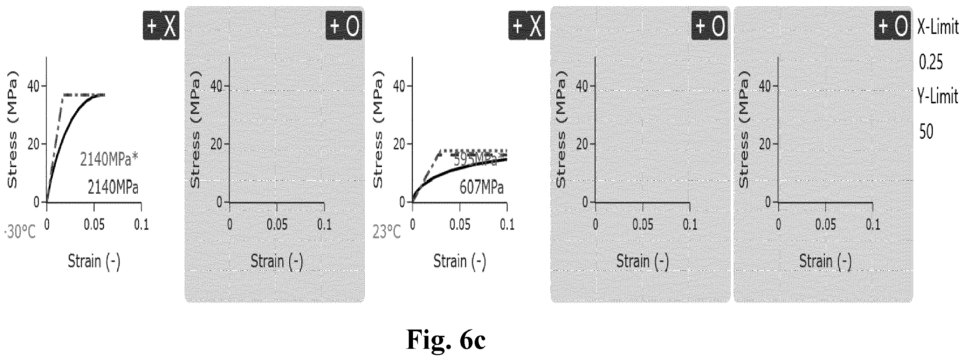

For materials A and B the respective experimental tensile curve is shown in Figs. 5c and 6c at temperatures of -30°C and 23°C.

The IPT, INC and TEN curves at different temperatures from -30°C to 50°C according to Figs. 5a-c and Figs. 6a-c are given as respective sets of input data values. Based on these sets of input data values, material properties at a wide range of temperatures and strain-rates can be extracted as explained above. Here, the term "material properties" should be understood as the set of constitutive output parameters listed in table 4 that define the thermo-viscoelastic-viscoplastic and failure response of the material.

Figs. 5a-b and Figs. 6a-b show the experimental force-displacement signals for the IPT and INC impact tests for the materials A and B respectively. The x and y coordinates of the dot symbol (•) correspond to the experimental limit load $F_{max}$ and the experimental deflection $U_{max}$ features respectively as inputted as set of input data 301. The x and y coordinates of the x-symbol (x) correspond to the predicted limit load $F_{max}$ and the predicted deflection $U_{max}$ features respectively as derived as output values 302.

The numerical values on the bottom-end of each chart in the Figs. 5a-b and Figs. 6a-b represent the magnitude of the force drop of a peak load $E_{br}$ feature, whereby the calibrated value is shown with a star symbol (*). The accuracy of the fit and the extrapolation capabilities of the invention can be evaluated for this example by comparing the location and magnitude of the experimental features with the location and magnitude of the predicted features. For the experimental curves at -30°C and +23°C, the result is a fit (best fit, because it has the smallest difference). However, for all other curves (0°C, +30°C and +50°C - shown with a greyed background), the result is in fact a prediction since these experimental curves were not used to extract the constitutive output parameters.

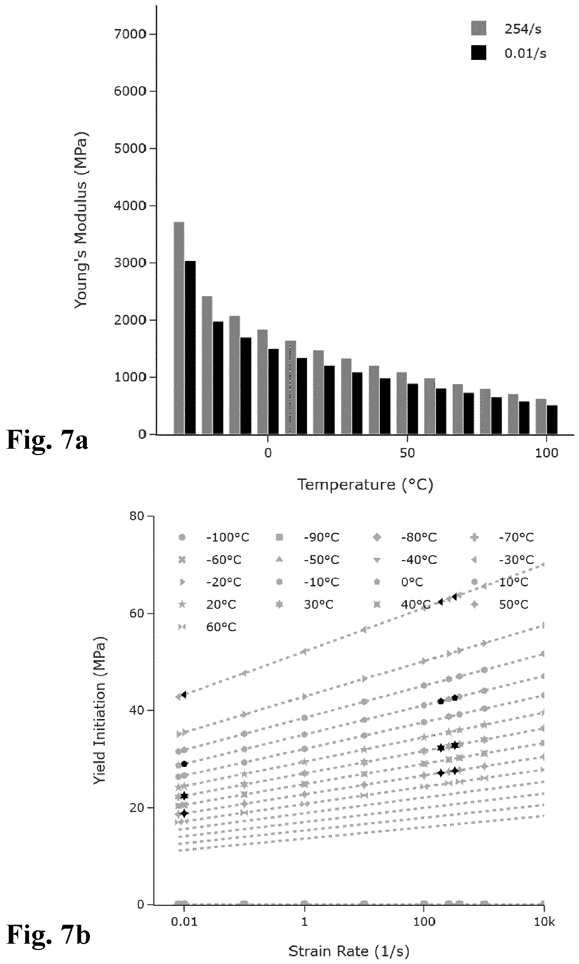

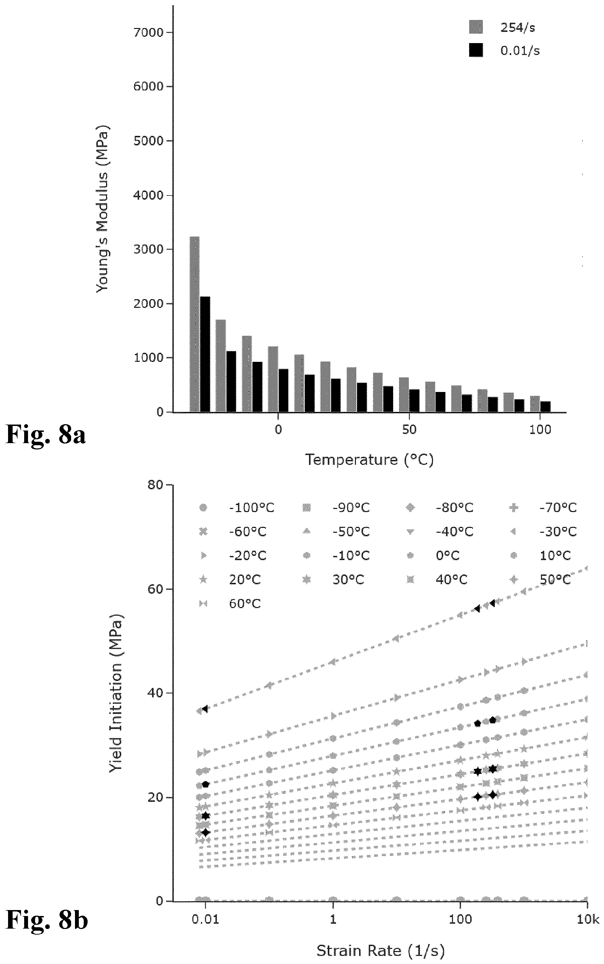

Figs. 7a to 7d illustrate output parameters representing material properties in graphical form for material A. Figs. 8a to 8d illustrate output parameters representing material properties in graphical form for material B. This information can be directly used to represent the thermo-viscoelastic-viscoplastic response with failure response of the material A or the material B when designing parts and components, typically using simulations via finite element methods.

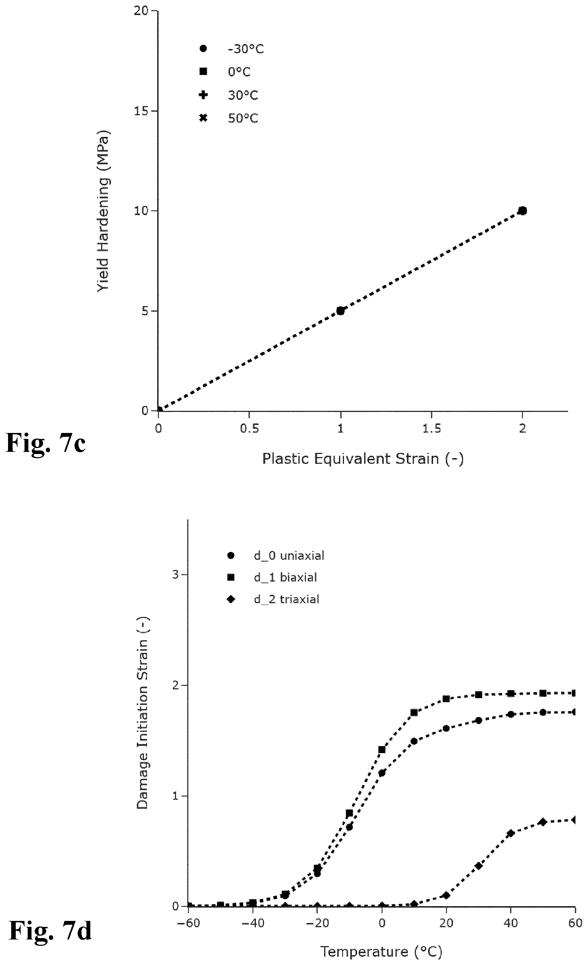

Fig. 7a illustrates the Young's modulus in MPa (elastic modulus) as a function of the temperature in degrees Celsius and the strain rate in 1/s for material A. Fig. 7b illustrates the Yield Initiation in MPa (yield stress) as a function of the temperature in degrees Celsius and the strain rate in 1/s for material A. Fig. 7c illustrates the Yield Hardening in MPa (hardening parameter) as a function of the Plastic Equivalent Strain for material A. Fig. 7d illustrates Damage Initiation strain (failure strain) as a function of the temperature in degrees Celsius and the state of stress/strain multiaxiality for material A.

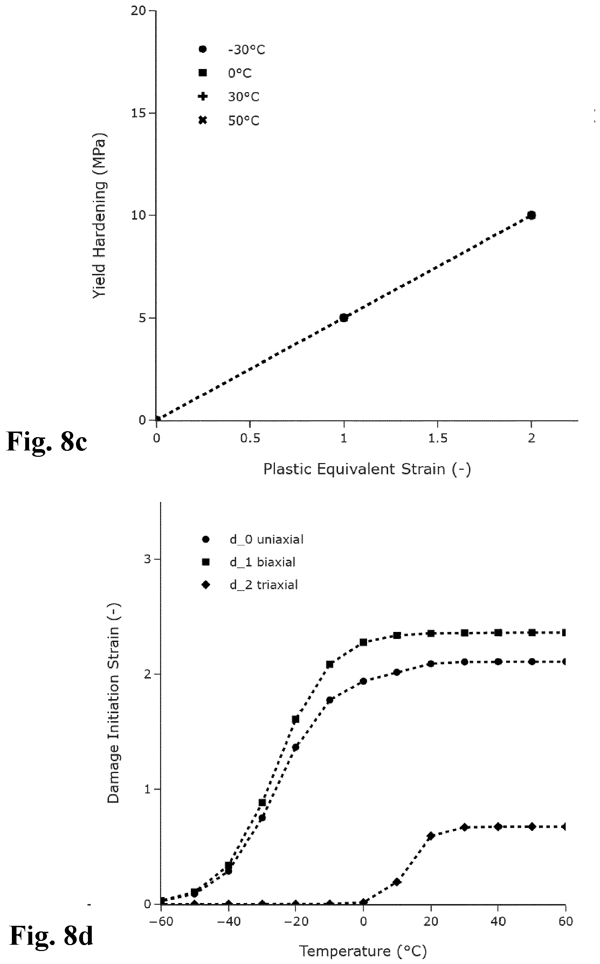

Fig. 8a illustrates the Young's modulus in MPa (elastic modulus) as a function of the temperature in degrees Celsius and the strain rate in 1/s for material B. Fig. 8b illustrates the Yield Initiation in MPa (yield stress) as a function of the temperature in degrees Celsius and the strain rate in 1/s for material B. Fig. 8c illustrates the Yield Hardening in MPa (hardening parameter) as a function of the Plastic Equivalent Strain for material B. Fig. 8d illustrates Damage Initiation strain (failure strain) as a function of the temperature in degrees Celsius and the state of stress/strain multiaxiality for material B.

The present disclosure includes methods and apparatuses which perform the methods described above, including data processing systems which perform these methods, and computer readable media containing instructions which when executed on data processing systems cause the systems to perform these methods.

Non-volatile memory can be a local device coupled directly to the rest of the components in the data processing system. A non-volatile memory that is remote from the system, such as a network storage device coupled to the data processing system through a network interface such as a modem or Ethernet interface, can also be used.

In the present disclosure, some functions and operations are described as being performed by or caused by software code to simplify description. However, such expressions are also used to specify that the functions result from execution of the code/instructions by a processor, such as a microprocessor.

Alternatively, or in combination, the functions and operations as described here can be implemented using special purpose circuitry, with or without software instructions, such as using Application-Specific Integrated Circuit (ASIC) or Field-Programmable Gate Array (FPGA). Embodiments can be implemented using hardwired circuitry without software instructions, or in combination with software instructions. Thus, the techniques are limited neither to any specific combination of hardware circuitry and software, nor to any particular source for the instructions executed by the data processing system.

While one embodiment can be implemented in fully functioning computers and computer systems, various embodiments are capable of being distributed as a computing product in a variety of forms and are capable of being applied regardless of the particular type of machine or computer-readable media used to actually effect the distribution.

At least some aspects disclosed can be embodied, at least in part, in software. That is, the techniques may be carried out in a computer system or other data processing system in response to its processor, such as a microprocessor, executing sequences of instructions contained in a memory, such as ROM, volatile RAM, non-volatile memory, cache or a remote storage device.

Routines executed to implement the embodiments may be implemented as part of an operating system or a specific application, component, program, object, module or sequence of instructions referred to as "computer programs." The computer programs typically include one or more instructions set at various times in various memory and storage devices in a computer, and that, when read and executed by one or more processors in a computer, cause the computer to perform operations necessary to execute elements involving the various aspects.

A machine-readable medium can be used to store software and data which when executed by a data processing system causes the system to perform various methods. The executable software and data may be stored in various places including for example ROM, volatile RAM, non-volatile memory and/or cache. Portions of this software and/or data may be stored in any one of these storage devices. Further, the data and instructions can be obtained from centralized servers or peer to peer networks. Different portions of the data and instructions can be obtained from different centralized servers and/or peer to peer networks at different times and in different communication sessions or in a same communication session. The data and instructions can be obtained in entirety prior to the execution of the applications. Alternatively, portions of the data and instructions can be obtained dynamically, just in time, when needed for execution. Thus, it is not required that the data and instructions be on a machine-readable medium in entirety at a particular instance of time.

Examples of computer-readable media include but are not limited to non-transitory, recordable, and non-recordable type media such as volatile and non-volatile memory devices, Read Only Memory (ROM), Random Access Memory (RAM), flash memory devices, floppy and other removable disks, magnetic disk storage media, optical storage media (e.g., Compact Disk Read-Only Memory (CD ROM), Digital Versatile Disks (DVDs), etc.), among others. The computer-readable media may store the instructions.

The instructions may also be embodied in digital and analog communication links for electrical, optical, acoustic or other forms of propagated signals, such as carrier waves, infrared signals, digital signals, etc. However, propagated signals, such as carrier waves, infrared signals, digital signals, etc. are not tangible machine readable medium and are not configured to store instructions.

In general, a machine-readable medium includes any mechanism that provides (i.e., stores and/or transmits) information in a form accessible by a machine (e.g., a computer, network device, personal digital assistant, manufacturing tool, any device with a set of one or more processors, etc.).

In various embodiments, hardwired circuitry may be used in combination with software instructions to implement the techniques. Thus, the techniques are neither limited to any specific combination of hardware circuitry and software nor to any source for the instructions executed by the data processing system.

The above description and drawings are illustrative and are not to be construed as limiting. Numerous specific details are described to provide a thorough understanding. However, in certain instances, well known or conventional details are not described in order to avoid obscuring the description. References to one or an embodiment in the present disclosure are not necessarily references to the same embodiment; and such references mean at least one.

In the foregoing specification, the disclosure has been described with reference to specific exemplary embodiments thereof. It will be evident that various modifications may be made thereto without departing from the broader scope as set forth in the following claims. The specification and drawings are, accordingly, to be regarded in an illustrative sense rather than a restrictive sense.

**Reference signs**

| | |
|---|---|
| 100 | Method |
| 101 - 109 | Method steps |
| 200 | Artificial neural network, ANN |
| 201 | Input neurons of ANN |
| 202 | Intermediate (hidden) neurons of ANN |
| 203 | Output neurons of ANN |
| 301 | Set of input data (values) |
| 302 | Set of output parameter (values) |
| 400 | Optimization scheme |
| 401 | Objective/fitness unit |
| IPT | Instrumented Impact Puncture Test |
| INC | Instrumented (V-notch) Charpy Test |
| TEN | Tensile Test |
| $F_{max}$ | Limit load |
| $U_{max}$ | Deflection at $F_{max}$ |
| $E_{br}$ | Peak load |
| $\sigma_y$. | Yield stress |

**Claims**

1. A computer-implemented method (100) for outputting parameter values describing at least an elastoplastic mechanical response of one or more materials, preferably of one or more polymers, the method comprising the following steps:

   • inputting (101), into an optimization algorithm (102), a set of data values of one or more experimental force-displacement signals derived in a mechanical impact test;
   • searching (103, 104), by means of the optimization algorithm (102), a combination of parameter values (301) of a material model representing one or more material properties, wherein this combination of parameter values (301) results in a difference between the set of data values inputted in the inputting step (101) and a prediction (107) of these parameter values, wherein the prediction is based on an estimation that is preferably performed by a trained artificial neural network (200), wherein the artificial neural network (200) is trained with one or more Finite Element simulations based on the one or more mechanical tests; and
   • outputting (108), from the optimization algorithm (102), parameter values that result in a predefined smallest difference between the set of data values inputted in the inputting step (101) and a finite element simulation or an estimation thereof performed by the artificial neural network (200).

2. The computer-implemented method (100) of claim 1, further comprise prior to the outputting step (108) the step of:

   • predicting (107) the one or more force-displacement signal by using the trained artificial neural network, wherein the artificial neural network comprises one or more neural processing units and mapping, by the trained artificial neural network, material properties representing a constitutive model to the set of data values based on one or more mechanical impact tests.

3. The computer-implemented method (100) of claim 1 or 2, wherein the output parameter values describe at least one strain-rate-dependent mechanical response and/or temperature dependent mechanical response, wherein preferably, the at least one strain-rate-dependent mechanical response and/or the at least one temperature dependent mechanical response is accounted in the optimization algorithm.

4. The computer-implemented method (100) of one of the preceding claims, wherein the trained artificial neural network (200) includes one or more feed forward multi-layer-perceptron neural network models, wherein for each mechanical test an own neural network model with its own hyperparameters is generated, each model having at least two hidden layers of neurons (202).

5. The computer-implemented method (100) of one of the preceding claims, wherein the one or more mechanical test is:

   • an instrumented Charpy impact test;
   • an instrumented Izod impact test;
   • an instrumented puncture test; and/or
   • an instrumented falling weight test/or
   • a tensile test at different temperatures.

6. The computer-implemented method (100) of one of the preceding claims, wherein the artificial neural network (200) is trained with data from a database of a plurality of finite element simulations to map one or more mechanical properties to a corresponding force-displacement signal.

7. The computer-implemented method (100) of one of the preceding claims, wherein the set of input data values is a set of numerical data in the form of at least one multi-point force-displacement signal, preferably representing one or more of an elasticity data, a plasticity data, a rate-dependent yield data, a damage data and/or a failure data.

8. The computer-implemented method (100) of one of the preceding claims, wherein prior to the inputting step, the method comprises a featurization step in which the number of input data values is reduced, wherein preferably one or more of following parameters represent the input data values:

   • a limit load ($F_{max}$);
   • a deflection ($U_{max}$);
   • a force drop of a peak load ($E_{br}$);
   • a slope ($E$) corresponding to least squares regression between 0.05% and 0.25% strain; and/or
   • stress at yield point ($\sigma_y$).

9. The computer-implemented method (100) of one of the preceding claims, wherein the material properties refer to a thermo-viscoelastic-viscoplastic model and wherein the parameter values is preferably one or more of:

   • a specific geometry;
   • a temperature;
   • a strain rate dependent stiffness;
   • a yield; and
   • a failure strain.

10. The computer-implemented method (100) of one of the preceding claims, wherein the outputted parameter values include at least one intrinsic material property, and the outputted parameter values describe features suitable for predicting the material property in conditions outside of the set of input data values.

11. The computer-implemented method (100) of one of the preceding claims, wherein the constitutive model is based on a non-linear model for elasticity or plasticity and preferably there is a failure parameter calibration.

12. The computer-implemented method of one of the preceding claims, wherein the constitutive model is one or more of the following models:

- a linear elasticity model, based on Hooke's law;
- a linear viscoelasticity model;
- a hyperelasticity model;
- a linear elastic isotropic strain hardening plasticity model;
- an ABAQUS perfect plasticity model;
- an ABAQUS isotropic hardening model;
- an ABAQUS kinematic hardening model;
- a Johnson-Cook isotropic hardening model;
- a Drucker-Prager model;
- a Mohr-Coulomb plasticity model;
- a crushable foam plasticity model;
- a Ramberg-Osgood plasticity model;
- a LS-DYNA MAT_24 model;
- an ABAQUS direct entry of test data model;
- an ABAQUS Two-layer viscoplasticity model;
- an ABAQUS yield stress ratios model;
- a Johnson-Cook rate dependence model;
- a Bergstrom-Boyce model;
- a Bergstrom Three Network model;
- a Bergstrom Hybrid model;
- a LS-DYNA SAMP-1 model;
- an ABAQUS progressive damage and failure for ductile metals model;
- an ABAQUS progressive damage and failure for fiber-reinforced materials model;
- a LS-DYNA model.

13. The computer-implemented method of one of the preceding claims, wherein the one or more experimental force-displacement signal was recorded during an impact loading experiment.

14. A non-transitory computer readable storage medium for tangibly storing computer program instructions capable of being executed by a processor, the computer program instructions defining the steps of one of the preceding method claims.

100

Fig. 1

200

**Fig. 2**

$$\left[ \begin{array}{cc} E & \bar{x}_1 \\ Y(\bar{\epsilon}_p = 0) & \bar{x}_2 \\ Y(\bar{\epsilon}_p = 2) & \bar{x}_3 \\ \bar{\epsilon}_p^D(\eta = 0.33) & \bar{x}_4 \\ \bar{\epsilon}_p^D(\eta = 0.66) & \bar{x}_5 \\ \bar{\epsilon}_p^D(\eta = 1.00) & \bar{x}_6 \\ \bar{u}_p^f & \bar{x}_7 \end{array} \right]$$

301     201

**Fig. 3a**

$$\left[ \begin{array}{cc} \bar{y}_1^* & f_{max} \\ \bar{y}_2^* & u_{max} \\ \bar{y}_3^* & E_{br} \end{array} \right]$$

203     302

**Fig. 3b**

400

Fig. 4

Fig. 5a

Impact

**Fig. 5b**

Tensile

**Fig. 5c**

EP 4 390 941 A1

Puncture

Fig. 6a

Impact

Fig. 6b

Fig. 6c

**Fig. 7a**

**Fig. 7b**

Fig. 7c

Fig. 7d

Fig. 8a

Fig. 8b

**Fig. 8c**

**Fig. 8d**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAUNDERS R. ET AL: "Mechanical behavior predictions of additively manufactured microstructures using functional Gaussian process surrogates", NPJ COMPUTATIONAL MATERIALS, vol. 7, no. 81, 3 June 2021 (2021-06-03), pages 1-11, XP093050179, DOI: 10.1038/s41524-021-00548-y Retrieved from the Internet: URL:https://www.nature.com/articles/s41524-021-00548-y.pdf> * the whole document * | 1-14 | INV. G16C60/00 |
| A | YIN M. ET AL: "Interfacing finite elements with deep neural operators for fast multiscale modeling of mechanics problems", COMPUTER METHODS IN APPLIED MECHANICS AND ENGINEERING, vol. 402, 10 May 2022 (2022-05-10), page 115027, XP093050330, AMSTERDAM, NL ISSN: 0045-7825, DOI: 10.1016/j.cma.2022.115027 * the whole document * | 1-14 | |
| A | ZHOU Y. ET AL: "Prediction of rupture and perforation limits of pressurised X80 pipelines using BP neural networks and generalised additive models", OCEAN ENGINEERING, PERGAMON, AMSTERDAM, NL, vol. 259, 6 July 2022 (2022-07-06), XP087151232, ISSN: 0029-8018, DOI: 10.1016/J.OCEANENG.2022.111839 [retrieved on 2022-07-06] * the whole document * | 1-14 | TECHNICAL FIELDS SEARCHED (IPC) G16C G06N |

-/--

The present search report has been drawn up for all claims

| Place of search | Date of completion of the search | Examiner |
|---|---|---|
| The Hague | 31 May 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

# EUROPEAN SEARCH REPORT

Application Number

EP 22 21 5219

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OULADBRAHIM A. ET AL: "Prediction of Gurson Damage Model Parameters Coupled with Hardening Law Identification of Steel X70 Pipeline Using Neural Network", METALS AND MATERIALS INTERNATIONAL, THE KOREAN INSTITUTE OF METALS AND MATERIALS, SEOUL, vol. 28, no. 2, 2 September 2021 (2021-09-02), pages 370-384, XP037680823, ISSN: 1598-9623, DOI: 10.1007/S12540-021-01024-4 [retrieved on 2021-09-02] * the whole document * | 1-14 | |
| A | DARABI M. K. ET AL: "A thermo-viscoelastic-viscoplastic-viscodamage constitutive model for asphaltic materials", INTERNATIONAL JOURNAL OF SOLIDS AND STRUCTURES, ELSEVIER, AMSTERDAM, NL, vol. 48, no. 1, 1 January 2011 (2011-01-01), pages 191-207, XP027453869, ISSN: 0020-7683, DOI: 10.1016/J.IJSOLSTR.2010.09.019 [retrieved on 2010-09-25] * the whole document * | 1-14 | TECHNICAL FIELDS SEARCHED (IPC) |

-/--

The present search report has been drawn up for all claims

| Place of search | Date of completion of the search | Examiner |
|---|---|---|
| The Hague | 31 May 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 22 21 5219

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YU X. ET AL: "Energy Separation and Explicit Dynamic Analysis of Low Temperature Impact Toughness of Transmission Tower Material Q420B", JOURNAL OF SHANGHAI JIAOTONG UNIVERSITY (ENGLISH EDITION), SHANGHAI JIAOTONG UNIVERSITY PRESS, HEIDELBERG, vol. 24, no. 3, 4 June 2019 (2019-06-04), pages 381-387, XP036798468, ISSN: 1007-1172, DOI: 10.1007/S12204-018-1963-4 [retrieved on 2019-06-04] * the whole document * | 1-14 | |
| A | STOLL A. ET AL: "Machine learning for material characterization with an application for predicting mechanical properties", GAMM-MITTEILUNGEN, vol. 44, no. 1, 1 March 2021 (2021-03-01), XP055942649, Hoboken, USA ISSN: 0936-7195, DOI: 10.1002/gamm.202100003 Retrieved from the Internet: URL:https://onlinelibrary.wiley.com/doi/full-xml/10.1002/gamm.202100003> * the whole document * | 1-14 | |
| A | US 9 274 036 B2 (UNIV KING FAHD PET & MINERALS [SA]) 1 March 2016 (2016-03-01) * the whole document * | 1-14 | TECHNICAL FIELDS SEARCHED (IPC) |
| A | US 2021/357555 A1 (LIU WING KAM [US] ET AL) 18 November 2021 (2021-11-18) * the whole document * | 1-14 | |

-/--

The present search report has been drawn up for all claims

| Place of search | Date of completion of the search | Examiner |
|---|---|---|
| The Hague | 31 May 2023 | Godzina, Przemyslaw |

EPO FORM 1503 03.82 (P04C01)

Europäisches
Patentamt

European
Patent Office

Office européen
des brevets

**EUROPEAN SEARCH REPORT**

Application Number

**EP 22 21 5219**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 631 647 B2 (SEALE JOSEPH B [US]) 14 October 2003 (2003-10-14) * the whole document * ----- | 1-14 | |
| A | US 9 348 056 B2 (BP CORP NORTH AMERICA INC [US]) 24 May 2016 (2016-05-24) * the whole document * ----- | 1-14 | |

TECHNICAL FIELDS
SEARCHED      (IPC)

The present search report has been drawn up for all claims

| Place of search | Date of completion of the search | Examiner |
|---|---|---|
| The Hague | 31 May 2023 | Godzina, Przemyslaw |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 4 of 4

## ANNEX TO THE EUROPEAN SEARCH REPORT
## ON EUROPEAN PATENT APPLICATION NO.

EP 22 21 5219

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2023

| Patent document cited in search report | | | Publication date | Patent family member(s) | | | Publication date |
|---|---|---|---|---|---|---|---|
| US 9274036 | B2 | | 01-03-2016 | NONE | | | |
| US 2021357555 | A1 | | 18-11-2021 | CN | 113168891 | A | 23-07-2021 |
| | | | | US | 2020089826 | A1 | 19-03-2020 |
| | | | | US | 2021357555 | A1 | 18-11-2021 |
| | | | | WO | 2020056405 | A1 | 19-03-2020 |
| US 6631647 | B2 | | 14-10-2003 | NONE | | | |
| US 9348056 | B2 | | 24-05-2016 | AU | 2014306018 | A1 | 12-11-2015 |
| | | | | CA | 2912191 | A1 | 12-02-2015 |
| | | | | CN | 105393110 | A | 09-03-2016 |
| | | | | EA | 201592039 | A1 | 29-02-2016 |
| | | | | EP | 3030888 | A1 | 15-06-2016 |
| | | | | JP | 6450385 | B2 | 09-01-2019 |
| | | | | JP | 2016532867 | A | 20-10-2016 |
| | | | | MX | 349507 | B | 02-08-2017 |
| | | | | US | 2015043787 | A1 | 12-02-2015 |
| | | | | WO | 2015021182 | A1 | 12-02-2015 |

EPO FORM P0459

For more details about this annex : see Official Journal of the European Patent Office, No. 12/82

# EP 4 390 941 A1

## REFERENCES CITED IN THE DESCRIPTION

### Patent documents cited in the description

- WO 2021110690 A1

- US 10955362 B2

### Non-patent literature cited in the description

- Analysis User's Guide, Part V: Materials. Dassault Systemes Simulia, Inc, 2016

- Material Models. KEYWORD USE''S MANUAL. Livermore Software Technology Corporation, 2021, vol. II